# EUROPÄISCHE PATENTSCHRIFT

(11) **EP 0 463 513 B1**
(45) Veröffentlichungstag und Bekanntmachung des Hinweises auf die Patenterteilung: **12.04.1995**
(21) Anmeldenummer: 91109806.9
(22) Anmeldetag: 14.06.1991
(51) Int. Cl.: C07C 69/738, C07C 251/48, C07C 67/343, A01N 37/42, A01N 37/50

(54) **Benzylketone und diese enthaltende Fungizide**
Benzylketones and fongicides containing them
Benzylecétones et fongicides les contenant

(30) Priorität: 27.06.1990 DE 4020397
(43) Veröffentlichungstag der Anmeldung: 02.01.1992
(73) Patentinhaber: BASF Aktiengesellschaft, 67063 Ludwigshafen (DE)
(72) Erfinder: Wingert, Horst, Dr., W-6800 Mannheim 1 (DE); Sauter, Hubert, Dr., W-6800 Mannheim 1 (DE); Roehl, Franz, Dr., W-6700 Ludwigshafen (DE); Doetzer, Reinhard, Dr., W-6940 Weinheim (DE); Lorenz, Gisela, Dr., W-6730 Neustadt (DE); Ammermann, Eberhard, Dr., W-6700 Ludwigshafen (DE)

(56) Entgegenhaltungen:
- EP-A- 0 253 213
- EP-A- 0 307 103
- EP-A- 0 342 459
- EP-A- 0 393 428

## Beschreibung

Die vorliegende Erfindung betrifft Benzylketone, Fungizide, die diese Verbindungen enthalten, und Verfahren zur Bekämpfung von Pilzen mit diesen Verbindungen.

Es ist bekannt, 2-(Phenoxymethyl)-phenylglyoxylsäure-methylester-O-methyloxim als Fungizid zu verwenden (EP 253 213). Die Verbindung läßt jedoch hinsichtlich ihrer fungiziden Wirkung zu wünschen übrig.

Der Erfindung lag die Aufgabe zugrunde, Wirkstoffe und Fungizide mit besserer Wirkung bereitzustellen.

Es wurde nun gefunden, daß Benzylketone der allgemeinen Formel I
in der
- X: CH oder N,
- Y: O oder NR⁴
- R¹, R²: H oder C₁-C₄-Alkyl
- R³: H, Halogen, Cyano, Nitro, C₁-C₆-Alkyl, C₃-C₆-Cycloalkyl, C₁-C₆-Alkoxy, Halogen-C₁-C₂-alkyl, Aryl-C₁-C₂-alkyl, Aryl-C₁-C₂-alkoxy, Aryloxy oder Aryl bedeutet, wobei die Arylgruppen ihrerseits durch einen bis zu drei der Reste Halogen, Cyano, Nitro, C₁-C₆-Alkyl, C₁-C₆-Alkoxy oder Halogen-C₁-C₂-alkyl substituiert sein können,
- R⁴: OH, C₁-C₆-Alkoxy oder Aryl-C₁-C₂-alkoxy und
- R⁵: H, Methyl, Halogen oder Methoxy bedeutet,
eine bessere fungizide Wirkung als die bekannten Wirkstoffe haben. Die Verbindungen haben auch eine insektizide Wirkung.

Von den Benzylketonen der allgemeinen Strukturformel I
sind diejenigen bevorzugt, bei denen die Substituenten die folgende Bedeutung haben:
- X: CH oder N,
- Y: O oder NR⁴
- R¹, R²: H oder C₁-C₄-Alkyl (z.B. Methyl, Ethyl, n-, iso-Propyl, n-, iso-, sec- und tert-Butyl),
- R³: H, Halogen (z.B. F, Cl, Br und J, Nitro, Cyano, C₁-C₆-Alkyl (z.B. Methyl, Ethyl, n-, iso-Propyl, n-, iso-, sec- und tert.-Butyl, n-Pentyl und n-Hexyl), C₃-C₆-Cycloalkyl (z.B. Cyclopropyl, Cyclobutyl, Cyclopentyl, Cyclohexyl), C₁-C₆-Alkoxy (z.B. Methoxy, Ethoxy, n-, iso-Propoxy, n-, iso-, sec- und tert.-Butoxy, Pentyloxy und Hexyloxy), Halogen-C₁-C₂-alkyl (z.B. Trifluormethyl, Trichlormethyl, Pentafluorethyl), Aryl-C₁-C₂-alkyl (z.B. Benzyl, Phenylethyl), Aryl-C₁-C₂-alkoxy (z.B. Benzyloxy, Phenylethoxy), Aryloxy (z.B. Phenoxy) und Aryl (z.B. Phenyl) bedeutet, wobei die Arylgruppe ihrerseits durch einen bis zu drei der Reste Halogen, Cyano, Nitro, C₁-C₆-Alkyl, C₁-C₆-Alkoxy oder Halogen-C₁-C₂-alkyl substituiert sein kann und
- R⁴: OH, C₁-C₆-Alkoxy oder Aryl-C₁-C₂-alkoxy und
- R⁵: Wasserstoff bedeutet.

Besonders bevorzugt sind Verbindungen der allgemeinen Strukturformel I, in der
- X: CH oder N
- Y: O
- R¹, R²: Methyl und
- R³: die oben angegebene Bedeutung hat und
- R⁵: Wasserstoff bedeutet.

Die neuen Verbindungen der allgemeinen Formel I können aufgrund der C=X-Doppelbindung sowohl als E- als auch als Z-Isomere vorliegen, die in üblicher Weise getrennt werden können. Sowohl die einzelnen isomeren Verbindungen, als auch ihre Gemische werden von der Erfindung umfaßt und sind als Fungizide brauchbar.

Man erhält die neuen Benzylketone I, mit Y=O beispielsweise dadurch, daß man ein Benzylhalogenid der allgemeinen Formel II in an sich bekannter Weise (vgl. Houben-Weyl Bd. 13/2 a S. 553 ff; P. Knochel, J. Org. Chem. 53, 2390 (1988)) in eine Zink-Kupfer-organische Verbindung der allgemeinen Formel III überführt und diese mit Carbonsäurehalogeniden der allgemeinen Formel IV zur Reaktion bringt (S.C. Berk, P. Knochel, M.C.P. Yeh, J. Org. Chem. 53, 5789 (1988)).
X, R¹, R² und R³ haben die gleiche Bedeutung wie in der Formel I, Y bedeutet O; Hal in Formel II, III ist Halogen, vorzugsweise Brom; Hal in Formel IV ist Halogen, vorzugsweise Chlor.

Die Benzylhalogenide II werden in der Regel bei Temperaturen von -5 bis 10°C, vorzugsweise bei 0°C mit aktiviertem Zink (P. Knochel, M.C.P. Yeh, S.C. Berk, J. Talbert, J. Org. Chem. 53, 2390, 1988) zur Reaktion gebracht. Anschließend erfolgt die Reaktion zur Kupfer/Zink-organischen Verbindung mit dem in Tetrahydrofuran löslichen CuCN·2LiCl-Komplex bei Temperaturen von -80 bis -10°C.

Die Umsetzung mit den Carbonsäurehalogeniden IV erfolgt z.B. in einem inerten, wasserfreien Lösungsmittel, wie z.B. Tetrahydrofuran bei Temperaturen von -70 bis 30°C.

Alternativ hierzu lassen sich die Verbindungen der allgemeinen Strukturformel I, mit Y=0 auch dadurch herstellen, daß man ein Benzylhalogenid der Formel II mit einem Carbonsäurehalogenid der Formel IV in Gegenwart von Zink und einem Übergangsmetall-Katalysator, wie z. B. Pd (Ph₃P)₄, PdCl₂, Pd (Ph₃P)₂Cl₂, Pd (PhCN)₂Cl₂, Pd (OAc)₂ oder Ni (PPh₃)₂Cl₂ in einem inerten Lösungmittel wie z.B. THF, Toluol oder bevorzugt Dimethoxyethan bei Temperaturen von -10 bis +100°C zur Reaktion bringt (vgl. T. Fujisawa et al., Chem. Lett., 1135 (1981)). Ph = Phenyl.
Die Ausgangsverbindungen II können aufgrund der C=X-Doppelbildung als E/Z-Isomerengemische oder aber als reine E- oder Z-Isomere vorliegen, so daß die entsprechenden Folgeprodukte, einschließlich der Benzylketone I, entweder als E/Z-Isomerengemische oder als E- oder Z-Isomere anfallen. Sowohl die einzelnen isomeren Verbindungen als auch ihre Gemische werden von der Erfindung umfaßt.

Die Darstellung der Ausgangsverbindungen II (X=CH) ist in EP 226917 beschrieben; die der Verbindungen II (X=N) in EP 354571.

Die Carbonsäurehalogenide der allgemeinen Formel IV (R³ hat die oben angegebene Bedeutung) sind entweder bekannt oder können durch Verfahren analog zu bekannten Verfahren hergestellt werden. Entsprechende Herstellvorschriften sind z.B. beschrieben in Houben-Weyl, Methoden der organischen Chemie, Bd. E5, S. 587.

Die Verbindungen der allgemeinen Formel I, wobei Y NR⁴ bedeutet, lassen sich aus den entsprechenden Verbindungen der Formel I, mit Y=0 herstellen, indem man diese in an sich bekannter Weise mit einem Amin der allgemeinen Struktur NH₂R⁴ (R⁴ hat die im Anspruch 1 angegebene Bedeutung) zur Reaktion bringt (vgl. Houben-Weyl, Methoden der organischen Chemie, Band X/4, S. 1).

Die folgenden Beispiele erläutern die Herstellung der Benzylketone.

### Beispiel 1

### Herstellung von 2-(2-Chlorphenylcarbonylmethyl)-phenyl-β-methoxyacrylsäure-methylester (Verb. 5, Tab. 2)

2.3 g Zinkpulver wird in 20 ml THF (Tetrahydrofuran) mit 700 mg 1,2-Dibromethan versetzt. Man erhitzt bis zum Ende der Gasentwicklung unter Rückfluß, läßt auf Raumtemperatur (20°C) abkühlen und gibt 0.2 ml Trimethylchlorsilan zu. Zu dieser Lösung tropft man bei 0°C eine Lösung von 10 g (35 mmol) α-(2-Brommethyl-phenyl)-β-methoxyacrylsäure-methylester in 40 ml THF und rührt 3 h bei 5°C nach. Anschließend wird auf -78°C abgekühlt und man gibt eine Lösung von 3.2 g Kupfercyanid und 3 g Lithiumchlorid in 40 ml THF zu. Man läßt 5 min auf -20°C auftauen, kühlt erneut auf -78°C ab und tropft eine Lösung von 6.1 g 2-Chlorbenzoylchlorid in 20 ml THF zu. Man rührt über Nacht bei Raumtemperatur. Der Reaktionsansatz wird auf Wasser gegossen, mit Essigester extrahiert, getrocknet und eingeengt. Chromatographie an Kieselgel mit Hexan/Essigester liefert 4.1 g der Verbindung Nr. 5, Tab. 2 als farblosen Feststoff mit einem Schmelzpunkt von 70-75°C. Ausbeute 35 %.

### Beispiel 2

### 2-(Phenylcarbonylmethyl)-phenyl-β-methoxyacrylsäure-methylester (Verb. 1, Tab. 2)

Unter Stickstoff tropft man zu 2.3 g (0.035 mol) Zinkpulver und 600 mg (0.9 mmol) Pd (Ph₃P)₂Cl₂ eine Lösung von 5 g (17.5 mmol) α-(2-Brommethylphenyl)-β-methoxyacrylsäure-methylester und 2.5 g (17.8 mmol) Benzoylchlorid in 50 ml Dimethoxyethan zu und erhitzt anschließend 30 min auf 50°C. Man versetzt mit Wasser, extrahiert mit Essigester, trocknet und engt ein. Der Rückstand wird an kieselgel mit Hexan/Essigester chromatographiert. Man erhält 1.6 g der Verbindung Nr. 1, Tab. 2 als hellgelben Feststoff mit einem Schmelzpunkt von 97-99°C.
Ausbeute 29 %.

In entsprechender Weise können die in den folgenden Tabellen aufgeführten Verbindungen hergestellt werden.

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g, je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:
I. Man vermischt 90 Gew.-Teile der Verbindung Nr. 20 (Tab. 3) mit 10 Gew.-Teilen N-Methyl-α-pyrrolidon und erhält eine Lösung, die zur Anwendung in Form kleinster Tropfen geeignet ist.
II. 20 Gew.-Teile der Verbindung Nr. 5 (Tab. 2) werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.
III. 20 Gew.-Teile der Verbindung Nr. 6 (Tab. 2) werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
IV. 20 Gew.-Teile der Verbindung Nr. 20 (Tab. 2) werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
V. 80 Gew.-Teile der Verbindung Nr. 20 (Tab. 3) werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.
VI. 3 Gew.-Teile der Verbindung Nr. 21 (Tab. 3) werden mit 97 Gew.-Teilen feinteiligem Kaolin innig vermischt. Man erhält auf diese Weise ein Stäubemittel, das 3 Gew.% des Wirkstoffs enthält.
VII. 30 Gew.-Teile der Verbindung Nr. 20 (Tab. 3) werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 40 Gew.-Teile der Verbindung Nr. 21 (Tab. 3) werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.
IX. 20 Gew.-Teile der Verbindung Nr. 20 (Tab. 2) werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

### Anwendungsbeispiele

Als Vergleichswirkstoff wurde 2-(Phenoxymethyl)-phenylglyoxylsäure-methylester-O-methyloxim (A) - bekannt aus EP 253 213 - verwendet.

### Anwendungsbeispiel 1

### Wirksamkeit gegen Botrytis cinerea

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22-24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß der Wirkstoff Nr. 20 (Tabelle 3) bei der Anwendung als 0,05 %ige (Gew.%) Spritzbrühe eine bessere fungizide Wirkung zeigt (55 %) als der bekannte Vergleichswirkstoff A (30 %).

### Anwendungsbeispiel 2

### Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20-22°C und 70 % relativer Luftfeuchtigkeit für weitere 5 Tage kultiviert. Dann wurde das Ausmaß des Blattbefalls ermittelt.

Das Ergebnis zeigt, daß die Wirkstoffe Nr. 5, 6 und 20 aus Tabelle 2 und Nr. 20 und 21 aus Tabelle 3 bei der Anwendung als 0,05 %ige Spritzbrühe eine bessere fungizide Wirkung zeigen (65 %) als der bekannte Vergleichswirkstoff A (30 %).

## Patentansprüche

1. Benzylketone der allgemeinen Formel I in der
X CH oder N,
Y O oder NR⁴
R¹, R² H oder C₁-C₄-Alkyl
R³ H, Halogen, Cyano, Nitro, C₁-C₆-Alkyl, C₃-C₆-Cycloalkyl, C₁-C₆-Alkoxy, Halogen-C₁-C₂-alkyl, Aryl-C₁-C₂-alkyl, Aryl-C₁-C₂-alkoxy, Aryloxy oder Aryl bedeutet, wobei die Arylgruppen ihrerseits durch einen bis zu drei der Reste Halogen, Cyano, Nitro, C₁-C₆-Alkyl, C₁-C₆-Alkoxy oder Halogen-C₁-C₂-alkyl substituiert sein können,
R⁴ OH, C₁-C₆-Alkoxy oder Aryl-C₁-C₂-alkoxy und
R⁵ H, Methyl, Halogen oder Methoxy bedeutet.

2. Fungizide Mittel, enthaltend einen inerten Trägerstoff und eine fungizid wirksame Menge einer Verbindung der Formel I in der
X CH oder N,
Y O oder NR⁴
R¹, R² H oder C₁-C₄-Alkyl
R³ H, Halogen, Cyano, Nitro, C₁-C₆-Alkyl, C₃-C₆-Cycloalkyl, C₁-C₆-Alkoxy, Halogen-C₁-C₂-alkyl, Aryl-C₁-C₂-alkyl, Aryl-C₁-C₂-alkoxy, Aryloxy oder Aryl bedeutet, wobei die Arylgruppen ihrerseits durch einen bis zu drei der Reste Halogen, Cyano, Nitro, C₁-C₆-Alkyl, C₁-C₆-Alkoxy oder Halogen-C₁-C₂-alkyl substituiert sein können,
R ⁴ OH, C₁-C₆-Alkoxy oder Aryl-C₁-C₂-alkoxy und
R⁵ H, Methyl, Halogen oder Methoxy bedeutet.

3. Verfahren zur Bekämpfung von Pilzen dadurch gekennzeichnet, daß man die Pilze oder die von Pilzbefall bedrohten Pflanzen, Samen, Materialien oder Flächen mit einer fungizid wirksamen Menge einer Verbindung der Formel I in der
X CH oder N,
Y O oder NR⁴
R¹, R² H oder C₁-C₄-Alkyl
R³ H, Halogen, Cyano, Nitro, C₁-C₆-Alkyl, C₃-C₆-Cycloalkyl, C₁-C₆-Alkoxy, Halogen-C₁-C₂-alkyl, Aryl-C₁-C₂-alkyl, Aryl-C₁-C₂-alkoxy, Aryloxy oder Aryl bedeutet, wobei die Aryl-gruppen ihrerseits durch einen bis zu drei der Reste Halogen, Cyano, Nitro, C₁-C₆-Alkyl, C₁-C₆-Alkoxy oder Halogen-C₁-C₂-alkyl substituiert sein können,
R⁴ OH, C₁-C₆-Alkoxy oder Aryl-C₁-C₂-alkoxy und
R⁵ H, Methyl, Halogen oder Methoxy bedeutet. behandelt.

4. Verbindung der Formel I gemäß Anspruch 1, in der R¹ und R² Methyl, Y O, X CH und R³ Wasserstoff bedeutet.

5. Verbindung der Formel I gemäß Anspruch 1, in der R¹ und R² Methyl, Y O, X N und R³ Methyl in 2-Stellung bedeutet.

6. Verbindung der Formel I gemäß Anspruch 1, in der R¹ und R² Methyl, Y O, X CH und R³ Methyl in 2-Stellung bedeutet.

7. Verfahren zur Herstellung der Benzylketone der allgemeinen Formel I in der
X CH oder N,
Y O oder NR⁴
R¹, R² H oder C₁-C₄-Alkyl
R³ H, Halogen, Cyano, Nitro, C₁-C₆-Alkyl, C₃-C₆-Cycloalkyl, C₁-C₆-Alkoxy, Halogen-C₁-C₂-alkyl, Aryl-C₁-C₂-alkyl, Aryl-C₁-C₂-alkoxy, Aryloxy oder Aryl bedeutet, wobei die Arylgruppen ihrerseits durch einen bis zu drei der Reste Halogen, Cyano, Nitro, C₁-C₆-Alkyl, C₁-C₆-Alkoxy oder Halogen-C₁-C₂-alkyl substituiert sein können,
R⁴ OH, C₁-C₆-Alkoxy oder Aryl-C₁-C₂-alkoxy und
R⁵ H, Methyl, Halogen oder Methoxy bedeutet,
dadurch gekennzeichnet, daß man ein Benzylhalogenid der allgemeinen Formel II mit Zink und CuCN·2LiCl in eine Zink-Kupfer-organische Verbindung der allgemeinen Formel III überführt und diese mit Carbonsäurehalogeniden der allgemeinen Formel IV umsetzt, X, R¹, R² und R³ haben die oben genannten Bedeutungen, Hal bedeutet Halogen, und die so erhaltenen Ketone gegebenenfalls mit einem Amin der allgemeinen Struktur NH₂R⁴, in der R⁴ die oben angegebene Bedeutung hat, zur Reaktion bringt.

## Claims

1. A benzyl ketone of the formula I where X is CH or N, Y is O or NR⁴, R¹ and R² are each H or C₁-C₄-alkyl, R³ is H, halogen, cyano, nitro, C₁-C₆-alkyl, C₃-C₆-cycloalkyl, C₁-C₆-alkoxy, halo-C₁- or -C₂-alkyl, aryl-C₁- or -C₂-alkyl, aryl-C₁- or -C₂-alkoxy, aryloxy or aryl, and the aryl groups in turn may be substituted by from one to three of the radicals halogen, cyano, nitro, C₁-C₆-alkyl, C₁-C₆-alkoxy or halo-C₁- or -C₂-alkyl, R⁴ is OH, C₁-C₆-alkoxy or aryl-C₁- or -C₂-alkoxy and R⁵ is H, methyl, halogen or methoxy.

2. A fungicide containing an inert carrier and a fungicidal amount of a compound of the formula I where X is CH or N, Y is O or NR⁴, R¹ and R² are each H or C₁-C₄-alkyl, R³ is H, halogen, cyano, nitro, C₁-C₆-alkyl, C₃-C₆-cycloalkyl, C₁-C₆-alkoxy, halo-C₁- or -C₂-alkyl, aryl-C₁- or -C₂-alkyl, aryl-C₁- or -C₂-alkoxy, aryloxy or aryl, and the aryl groups in turn may be substituted by from one to three of the radicals halogen, cyano, nitro, C₁-C₆-alkyl, C₁-C₆-alkoxy or halo-C₁- or -C₂-alkyl, R⁴ is OH, C₁-C₆-alkoxy or aryl-C₁- or -C₂-alkoxy and R⁵ is H, methyl, halogen or methoxy.

3. A method for controlling fungi, wherein the fungi or the plants, seeds, materials or areas threatened by fungal attack are treated with a fungicidal amount of a compound of the formula I where X is CH or N, Y is O or NR⁴, R¹ and R² are each H or C₁-C₄-alkyl, R³ is H, halogen, cyano, nitro, C₁-C₆-alkyl, C₃-C₆-cycloalkyl, C₁-C₆-alkoxy, halo-C₁- or -C₂-alkyl, aryl-C₁- or -C₂-alkyl, aryl-C₁- or -C₂-alkoxy, aryloxy or aryl, and the aryl groups in turn may be substituted by from one to three of the radicals halogen, cyano, nitro, C₁-C₆-alkyl, C₁-C₆-alkoxy or halo-C₁- or -C₂-alkyl, R⁴ is OH, C₁-C₆-alkoxy or aryl-C₁- or -C₂-alkoxy and R⁵ is H, methyl, halogen or methoxy.

4. A compound of the formula I as claimed in claim 1, wherein R¹ and R² are each methyl, Y is O, X is CH and R³ is hydrogen.

5. A compound of the formula I as claimed in claim 1, wherein R¹ and R² are each methyl, Y is O, X is N and R³ is methyl in the 2-position.

6. A compound of the formula I as claimed in claim 1, wherein R¹ and R² are each methyl, Y is O, X is CH and R³ is methyl in the 2-position.

7. A process for the preparation of a benzyl ketone of the formula I where X is CH or N, Y is O or NR⁴, R¹ and R² are each H or C₁-C₄-alkyl, R³ is H, halogen, cyano, nitro, C₁-C₆-alkyl, C₃-C₆-cycloalkyl, C₁-C₆-alkoxy, halo-C₁- or -C₂-alkyl, aryl-C₁- or -C₂-alkyl, aryl-C₁- or -C₂-alkoxy, aryloxy or aryl, and the aryl groups in turn may be substituted by from one to three of the radicals halogen, cyano, nitro, C₁-C₆-alkyl, C₁-C₆-alkoxy or halo-C₁- or -C₂-alkyl, R⁴ is OH, C₁-C₆-alkoxy or aryl-C₁- or -C₂-alkoxy and R⁵ is H, methyl, halogen or methoxy, wherein a benzyl halide of the formula II is converted with zinc and CuCN·2LiCl into an organo-zinc-copper compound of the formula III and the latter is reacted with a carbonyl halide of the formula IV where X, R¹, R² and R³ have the abovementioned meanings and Hal is halogen, and the ketone thus obtained is, if required, reacted with an amine of the structure NH₂R⁴, where R⁴ has the abovementioned meanings.

## Revendications

1. Benzylcétones de formule générale I dans laquelle
X représente CH ou N,
Y représente O ou NR⁴,
R¹, R² représentent H ou un groupe en C1-C4;
R³ représente H, un halogène, un groupe cyano, nitro, alkyle en C1-C6, cycloalkyle en C3-C6, alcoxy en C1-C6, halogénoalkyle en C1-C2, aryl-alkyle en C1-C2, aryl-alcoxy en C1-C2, aryloxy ou aryle, les groupes aryle pouvant eux-mêmes porter un à trois substituants choisis parmi les halogènes, les groupes cyano, nitro, alkyle en C1-C6, alcoxy en C1-C6 ou halogénoalkyle en C1-C2,
R⁴ représente OH, un groupe alcoxy en C1-C6 ou aryl-alcoxy en C1-C2 et
R⁵ représente H, un groupe méthyle, un halogène ou un groupe méthoxy.

2. Produits fongicides contenant un véhicule inerte et une quantité fongicide efficace d'un composé de formule I dans laquelle
X représente CH ou N,
Y représente O ou NR⁴,
R¹, R² représentent H ou un groupe alkyle en C1-C4,
R³ représente H, un halogène, un groupe cyano, nitro, alkyle en C1-C6, cycloalkyle en C3-C6, alcoxy en C1-C6, halogénoalkyle en C1-C2, aryl-alkyle en C1-C2, aryl-alcoxy en C1-C2, aryloxy ou aryle, les groupes aryle pouvant eux-mêmes porter un à trois substituants choisis parmi les halogènes, les groupes cyano, nitro, alkyle en C1-C6, alcoxy en C1-C6 ou halogénoalkyle en C1-C2,
R⁴ représente OH, un groupe alcoxy en C1-C6 ou aryl-alcoxy en C1-C2 et
R⁵ représente H, un groupe méthyle, un halogène ou un groupe méthoxy.

3. Procédé pour combattre les mycètes, caractérisé en ce que l'on traite les mycètes ou les végétaux, semences, matériaux ou surfaces menacés d'une attaque par les mycètes, par une quantité fongicide efficace d'un composé de formule I dans laquelle
X représente CH ou N,
Y représente O ou NR⁴,
R¹, R² représentent H ou un groupe alkyle en C1-C4,
R³ représente H, un halogène, un groupe cyano, nitro, alkyle en C1-C6, cycloalkyle en C3-C6, alcoxy en C1-C6, halogénoalkyle en C1-C2, aryl-alkyle en C1-C2, aryl-alcoxy en C1-C2, aryloxy ou aryle, les groupes aryle pouvant eux-mêmes porter un à trois substituants choisis parmi les halogènes, les groupes cyano, nitro, alkyle en C1-C6, alcoxy en C1-C6 ou halogénoalkyle en C1-C2,
R⁴ représente OH, un groupe alcoxy en C1-C6 ou aryl-alcoxy en C1-C2 et
R⁵ représente H, un groupe méthyle, un halogène ou un groupe méthoxy.

4. Composé de formule I selon la revendication 1, dans laquelle R¹ et R² représentent des groupes méthyle, Y représente O, X représente CH et R³ l'hydrogène.

5. Composé de formule I selon la revendication 1, dans laquelle R¹ et R² représentent des groupes méthyle, Y représente O, X représente N et R³ un groupe méthyle en position 2.

6. Composé de formule I selon la revendication 1, dans laquelle R¹ et R² représentent des groupes méthyle, Y représente O, X représente CH et R³ un groupe méthyle en position 2.

7. Procédé de préparation des benzylcétones de formule générale I dans laquelle
X représente CH ou N,
Y représente O ou NR⁴,
R¹, R² représentent H ou un groupe alkyle en C1-C4,
R³ représente H, un halogène, un groupe cyano, nitro, alkyle en C1-C6, cycloalkyle en C3-C6, alcoxy en C1-C6, halogénoalkyle en C1-C2, aryl-alkyle en C1-C2, aryl-alcoxy en C1-C2, aryloxy ou aryle, les groupes aryle pouvant eux-mêmes porter un à trois substituants choisis parmi les halogènes, les groupes cyano, nitro, alkyle en C1-C6, alcoxy en C1-C6 ou halogénoalkyle en C1-C2,
R⁴ représente OH, un groupe alcoxy en C1-C6 ou aryl-alcoxy en C1-C2 et
R⁵ représente H, un groupe méthyle, un halogène ou un groupe méthoxy,
caractérisé en ce que l'on convertit un halogénure de benzyle de formule générale II, à l'aide du zinc et de CuCN.2LiCl, en un composé organique de zinc-cuivre de formule générale III qu'on fait ensuite réagir avec les halogénures d'acides carboxyliques de formule IV X, R¹, R² et R³ ayant les significations indiquées ci-dessus, Hal représentant un halogéne, et le cas échéant on fait réagir les cétones ainsi obtenues avec une amine de structure générale NH₂R⁴ dans laquelle R⁴ a les significations indiquées ci-dessus.
